# EUROPEAN PATENT SPECIFICATION

(11) **EP 0 787 474 B1**
(45) Date of publication and mention of the grant of the patent: **24.07.2002**
(21) Application number: 97870009.4
(22) Date of filing: 29.01.1997
(51) Int. Cl.: A61F 13/15

(54) **Disposable absorbent undergarment of pants type**
Wegwerfhöschen
Slip absorbant jetable

(30) Priority: 31.01.1996 JP 1599596
(43) Date of publication of application: 06.08.1997
(73) Proprietor: Uni-Charm Corporation, Kawanoe-shi, Ehime-ku (JP)
(72) Inventor: Hisada, Kenichi, Kawanoe-shi, Ehime-ken (JP)
(74) Representative: Van Malderen, Joelle

(56) References cited:
- EP-A- 0 487 921
- WO-A-93/17648

## Description

The present invention relates generally to disposable absorbent undergarments of pants type suitable to be used in the form of a disposable diaper, training pants, incontinence pants or the like.

Conventional disposable diapers of pants type are generally provided along an edge of a waist-opening thereof with circumferentially extending elastic members. Specifically, Japanese Patent Publication Application No. Hei7-44945 discloses a disposable diaper provided along edges of an waist-opening defined by front and rear waist regions thereof with a first group of elastic members circumferentially extending parallel one to another and, in addition, on the front and rear waist regions between the firt group and a crotch region thereof with a second group of elastic members circumferentially extending paralell one to another. In this known diaper, opposite side edges of the front and rear waist regions are put flat and joined together, respectively, to form the diaper of pants type. Consequently, the elastic members on the front and rear waist regions substantially overlap at the side edges put flat and bonded together and the respective circumferential lines of elasticity are continuous.

Locations of each side edge at which the elastic members of the front and rear waist regions overlap are substantially thicker than the remaining locations of this side edge and present the correspondingly high rigidity. As a result, the locations at which the elastic members of the front and rear waist regions overlap, form protuberances which are relatively stiff and deteriorate a touch of the diaper.

Document WO 93/17648 discloses a pants type diaper, comprising a front-part, a back-part, a crotch-part between the front and back parts, and an absorbent pad, wherein at least one of front and back parts has at least one elastically stretchable region. Preferably, the stretchable region is stretchable essentially in the transverse direction of the absorbent pad. The stretchable region may include stretchable elements, for instance stretchable threads, bands, ribbons or the like, which are mounted in a pre-stretched state.

In view of the problem as has been described above, it is a principal object of the invention to provide a disposable absorbent undergarment of pants type provided on front and rear waist regions thereof with circumferentially extending elastic members wherein the elastic members are arranged so as to eliminate an apprehension that the elastic members might cause a touch of the diaper particularly at transversely opposite side edges of the diaper to be deteriorated.

The object set forth above is achieved, according to the invention, by a disposable absorbent undergarment of pants type having a longitudinal axis, a front region, a rear region and a crotch region therebetween, the undergarment comprising a liquid-permeable topsheet, a liquid-impermeable backsheet and a liquid-absorbent core disposed therebetween, the front and rear waist regions being put flat with the topsheet being located inside and bonded together along transversely opposite side edges thereof to define a waist-opening and a pair of leg-openings and provided with a plurality of elastic members circumferentially extending parallel one to another and spaced apart one from another along the longitudinal axis, wherein:
the elastic members comprise an arrangement such that, between each pair of adjacent elastic members provided on one of the front and rear waist regions, each of the elastic members provided on the other of the front and rear waist regions is interposed.

The invention allows the disposable undergarment of pants type to offer its side edges of relatively soft and comfortable touch even when the mutually facing side edges of the front and rear waist regions are put flat and bonded together since the elastic members circumferentially extending on the front and rear waist regions alternate one with another longitudinally of the diaper without being overlapped one by another. Furthermore, the respective side edges of the front and rear waist regions present relative even and planar surfaces since along these side edges the elastic members are never overlapped one by another. This feature contributes to facilitate the side edges to be joined together, for example, by the ultrasonic-seaming.
Fig. 1 is a perspective view showing the inventive undergarment (diaper) as partially broken away;
Fig. 2 is a plan view showing the diaper as developed longitudinally thereof;
Fig. 3 is a plan view of the diaper shown by Fig. 1 as partially broken away;
Fig. 4 is a view similar to Fig. 3 showing another embodiment of the inventive diaper.

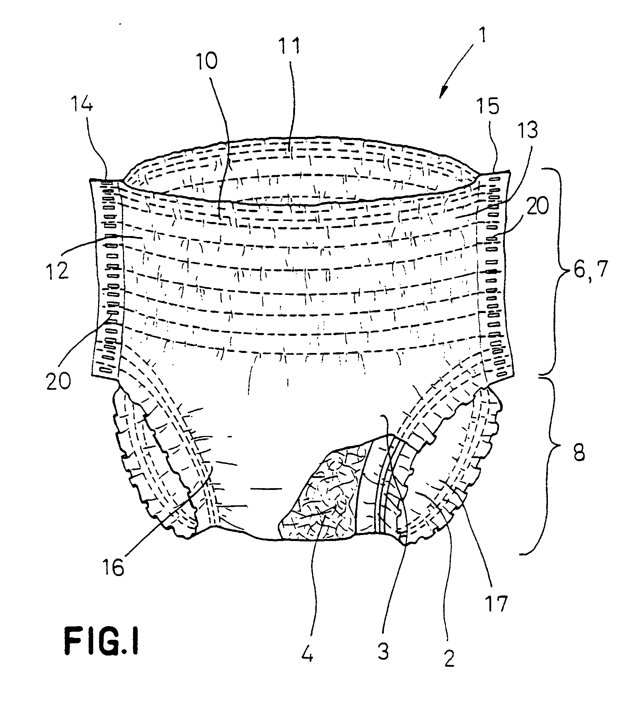

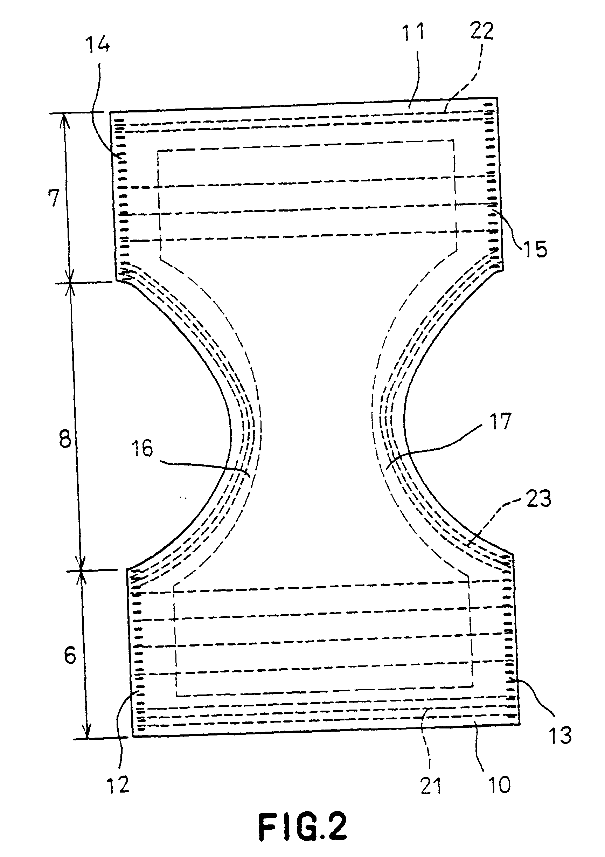

Figs. 1 and 2 respectively illustrate a diaper 1 of pants type as an embodiment of the invention in a perspective view as partially broken away and in a plan view as opened along transversely opposite side edges thereof and longitudinally developed. The diaper 1 is elongate along a longitudinal axis thereof in the developed condition and has a front waist region 6, a rear waist region 7 and a crotch region 8 extending therebetween. The diaper 1 typically comprises a liquid-permeable topsheet 2, a liquid-impermeable backsheet 3 and a liquid-absorbent core 4 disposed therebetween. The topsheet 2 and the backsheet 3 are joined together along portions thereof extending outward beyond a peripheral edge of the core 4 so as to define longitudinally opposite ends 10, 11, transversely opposite side edges 12, 13; 14, 15 of the front and rear waist regions 6, 7 and a pair of inwardly curved edges 16, 17 in the crotch region 8 adapted to surround the wearer's legs.

Referring to Fig. 2, the front and rear waist regions 6, 7 respectively include a plurality of elastic members 21, 22 extending along longitudinally opposite ends 10, 11 thereof and spaced apart one from another longitudinally of the diaper 1 and a plurality of elastic members 23, 23 extending along the respective inwardly curved edges 16, 17. The elastic members 21 - 23 are disposed between the topsheet 2 and the backsheet 3 or between the backsheet 3 and the core 4 so as to be secured to these two sheets 2, 3 or to the core 4. From the state shown by Fig. 2, the diaper 1 is folded in two with the topsheet 2 being located inside, then the respective side edges 12, 14 of the front and rear waist regions 6, 7 are ultrasonic-seamed to each other at intermittent spots 20 (See Fig. 1) and similarly the respective side edges 13, 15 are ultrasonic-seamed to each other and a waist-opening and a pair of leg-openings are thereby defined as shown in Fig. 1.

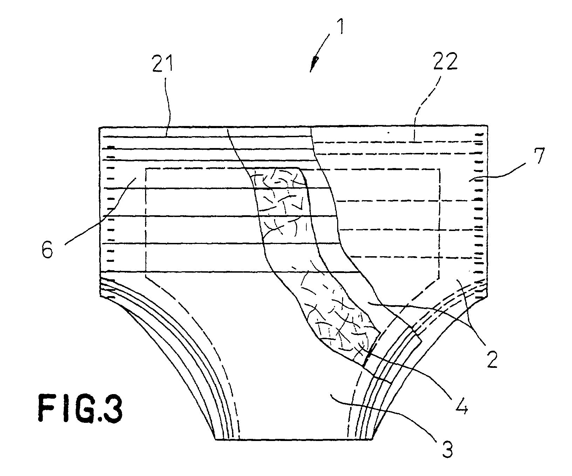

Fig. 3 is a schematic plan view partially broken away of the same diaper as that shown in Fig. 1, exemplarily illustrating how the elastic members 21, 22 on the respective waist regions are arranged. According to the embodiment illustrated in Fig. 3, the elastic members 21 on the front waist region 6 are indicated by solid lines and the elastic members 22 on the rear waist region 7 are indicated by broken lines. A plurality of elastic members 21 transversely extend parallel one to another and a plurality of elastic members 22 also transversely extend parallel to one to another but alternate with the plurality of elastic members 21, respectively. The respective elastic members 21, 22 arranged in this manner are not overlapped by each other as the diaper 1 is folded in two and thereby the side edges 12, 14; 13, 15 of the diaper can be maintained in relatively planar and smooth not only before but also after being subjected to a process of the ultrasonic-seaming. The side edges 12, 14; 13, 15 which are planar prior to the ultrasonic-seaming facilitate the mutually facing side edges to be closely placed one upon another.

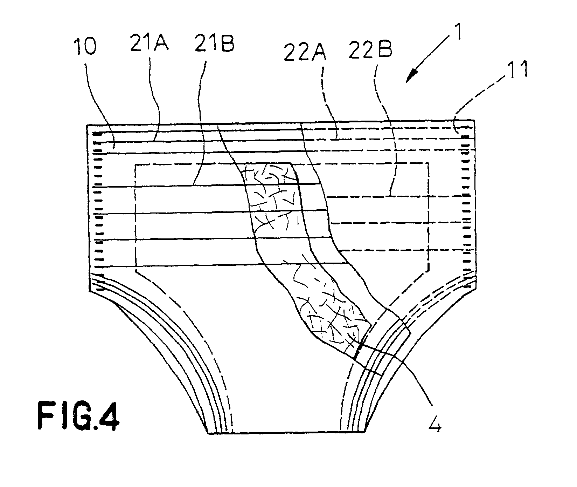

Fig. 4 is a view similar to Fig. 3 showing another embodiment of the invention. With the diaper 1 according to this embodiment, the elastic members 21A, 22A extending along the longitudinally opposite ends 10, 11 of the diaper 1 are overlapped one by another but the elastic members 21B, 22B extending across the core 4 alternate one with another longitudinally of the diaper 1.

To implement the invention, nonwoven fabric, perforated plastic film or the like may be used as material for the topsheet 2 and nonwoven fabric, plastic film or the like may be used as material for the backsheet 3 of the diaper 1. The liquid-absorbent core 4 may be formed, for example, by fluff pulp or a mixture of such fluff pulp and superabsorptive particles. The respective elastic members 21, 22, 23 may be selected from those having desired cross-sectional shapes and elasticities and securing of them to the topsheet 2 and the backsheet 3 may be preferably achieved by use of hot melt type adhesive. For joining or bonding of the remaining components of the diaper 1 may be achieved by, in addition to use of appropriate adhesive agents such as hot melt type adhesive, the heat-sealing technique so far as the components to be bonded are of heat-sealable nature.

## Claims

1. A disposable absorbent undergarment of pants type (1) having a longitudinal axis, a front region (6), a rear region (7) and a crotch region (8) therebetween, the undergarment comprising a liquid-permeable topsheet (2), a liquid-impermeable backsheet (3) and a liquid-absorbent core (4) disposed therebetween, the front (6) and rear (7) waist regions being put flat with the topsheet being located inside and joined together along transversely opposite side edges (12, 13, 14, 15) thereof to define a waist-opening and a pair of leg-openings and provided with a plurality of elastic members (21, 22) circumferentially extending parallel one to another and spaced apart one from another along the longitudinal axis, ***characterised in that*** the elastic members (21, 22) comprise an arrangement such that, between each pair of adjacent elastic members (21A, 21B; 22A, 22B) provided on one of the front (6) and rear waist (7) regions, each of the elastic members provided on the other of the front (6) and rear waist (7) regions is interposed.

2. The undergarment according to claim 1, wherein the arrangement of the elastic members is adopted for the elastic members (21, 22, 21A, 22A) lying within regions defined between longitudinally opposite ends (30, 31) of the front and rear regions (6, 7) and longitudinally opposite ends (32, 33) of the core (4) respectively.

3. The undergarment according to any of the preceding claims, wherein the arrangement of the elastic member is adopted for the elastic members (21B, 22B) extending across the liquid-absorbent core (4).

4. The undergarment according to any of the preceding claims, wherein each of the elastic members (21-22A) lying within regions defined between longitudinally opposite ends (30, 31) of the front and rear regions (6, 7) and longitudinally opposite ends of the core (4) respectively, has a relative width and thickness and a relatively high elasticity, and each of the elastic members (21B, 22B) extending across the liquid-absorbent core (4) has a relative narrowness and thickness and a relatively low elasticity.

## Patentansprüche

1. Absorbierendes Wegwerfhöschen (1) des Unterwäschetyps, das eine Längsachse, eine Vorderseite (6), eine Hinterseite (7) und einen Schrittteil (8) zwischen denselben aufweist, wobei das Unterhöschen ein flüssigkeitsdurchlässiges Obertuch (2), ein flüssigkeitsundurchlässiges Hintertuch (3) und einen dazwischen angeordneten flüssigkeitsabsorbierenden Kern (4) umfaßt, wobei die Hüftenbereiche der Vorderseite (6) und der Hinterseite (7) mit dem Obertuch, das sich im Innern befindet, flach aufeinander gelegt sind und quer entlang den entgegengesetzten Seitenkanten (12, 13, 14, 15) miteinander verbunden sind, um dadurch eine Hüftenöffnung und ein Paar von Öffnungen für die Beine zu definieren, und mit einer Vielzahl von elastischen Elementen (21, 22) versehen sind, die sich am Umfang parallel das eine zu dem anderen erstrecken und entlang der Längsachse im Abstand das eine von dem anderen angeordnet sind, **wobei** die elastischen Elemente (21, 22) eine derartige Anordnung umfassen, daß zwischen einem jeden Paar der aneinander grenzenden elastischen Elemente (21A, 21B; 22A, 22B), die an einem der Hüftbereiche der Vorderseite (6) und der Hinterseite (7) vorgesehen sind, ein jedes der elastischen Elemente, die auf den anderen Hüftbereichen der Vorderseite (6) und der Hinterseite (7) vorgesehen sind, dazwischen gelegt ist.

2. Höschen gemäß Anspruch 1, bei welchem die Anordnung der elastischen Elemente für die elastischen Elemente (21, 22, 21A, 22A) gewählt wird, die sich innerhalb der Bereiche befinden, die zwischen den längsseitig entgegengesetzten Enden (30, 31) der Vorder- und Hinterbereiche (6, 7) und den jeweiligen längsseitig entgegengesetzten Enden (32, 33) des Kerns (4) definiert sind.

3. Höschen gemäß irgendeinem der vorstehenden Ansprüche, bei welchem die Anordnung des elastischen Elementes für die elastischen Elemente (21B, 22B) gewählt wird, die sich quer über den flüssigkeitsabsorbierenden Kern (4) erstrecken.

4. Höschen gemäß irgendeinem der vorstehenden Ansprüche, bei welchem ein jedes der elastischen Elemente (21 - 22A), die innerhalb den Bereichen liegen, die zwischen den längsseitig entgegengesetzten Enden (30, 31) der Vorder- und Hinterbereiche (6, 7) und den jeweiligen längsseitig entgegengesetzten Enden des Kerns (4) definiert sind, eine relative Breite und Dicke sowie eine relativ hohe Elastizität aufweist und ein jedes der elastischen Elemente (21B, 22B), die sich quer über den flüssigkeitsabsorbierenden Kern (4) erstrecken, eine relative Enge und Dicke sowie eine relativ geringe Elastizität aufweist.

## Revendications

1. Sous-vêtement absorbant jetable du type culotte (1) ayant un axe longitudinal, une région avant (6), une région arrière (7) et une région d'entrejambes (8) entre celles-ci, le sous-vêtement comprenant une feuille supérieure perméable aux liquides (2), une feuille inférieure imperméable aux liquides (3) et un noyau absorbant les liquides (4) disposé entre celles-ci, les régions de ceinture avant (6) et arrière (7) étant déployées à plat avec la feuille supérieure à l'intérieur et jointes ensemble le long de leurs bords latéraux transversalement opposés (12, 13, 14, 15) pour définir une ouverture de taille et une paire d'ouvertures de jambes et étant pourvues d'une pluralité d'éléments élastiques (21, 22) s'étendant périphériquement et parallèlement à distance l'une de l'autre le long de l'axe longitudinal, **caractérisé en ce que** les éléments élastiques (21, 22) sont arrangés de façon telle que chacun des éléments élastiques prévus sur l'une des régions de ceinture avant (6) et arrière (7) soit intercalé entre chaque paire d'éléments élastiques adjacents (21A, 21B ; 22A, 22B) prévue sur l'autre des régions de ceinture avant (6) et arrière (7).

2. Sous-vêtement selon la revendication 1, dans lequel l'aménagement des éléments élastiques est adopté pour les éléments élastiques (21, 22, 21A, 22A) se trouvant dans des régions définies respectivement entre des extrémités longitudinalement opposées (30, 31) des régions avant et arrière (6, 7) et des extrémités longitudinalement opposées (32, 33) du noyau (4).

3. Sous-vêtement selon l'une quelconque des revendications précédentes, dans lequel l'aménagement des éléments élastiques est adopté pour les éléments élastiques (21B, 22B) s'étendant en travers du noyau absorbant les liquides (4).

4. Sous-vêtement selon l'une quelconque des revendications précédentes, dans lequel chacun des éléments élastiques (21, 22A) se trouvant dans des régions définies respectivement entre les extrémités longitudinalement opposées (30, 31) des régions avant et arrière (6, 7) et les extrémités longitudinalement opposées du noyau (4) a une largeur et une épaisseur relatives et une élasticité relativement élevée et chacun des éléments élastiques (21B, 22B) s'étendant en travers du noyau absorbant les liquides (4) a une étroitesse et une épaisseur relatives et une élasticité relativement basse.
